# FASCICULE DE BREVET EUROPEEN

(11) **EP 1 430 022 B1**
(45) Date de publication et mention de la délivrance du brevet: **31.03.2010**
(21) Numéro de dépôt: 02730374.2
(22) Date de dépôt: 29.04.2002
(51) Int. Cl.: C07C 253/30, C07C 255/65

(54) **PROCEDE DE PREPARATION DE COMPOSES TYPE AZOIQUE**
VERFAHREN ZUR HERSTELLUNG VON AZOVERBINDUNGEN
METHOD FOR PRODUCTION OF AZO COMPOUNDS

(30) Priorité: 28.06.2001 FR 0108541
(43) Date de publication de la demande: 23.06.2004
(73) Titulaire: ARKEMA FRANCE, 92700 Colombes (FR)
(72) Inventeur: BOSSOUTROT, Jean-Michel, F-69630 Chaponost (FR)
(74) Mandataire: Dang, Doris
(86) Numéro de dépôt international: PCT/FR2002/001488
(87) Numéro de publication internationale: WO 2003/002521

(56) Documents cités:
- WO-A-00/24706
- US-A- 2 469 358
- US-A- 2 586 995

## Description

La présente invention concerne un procédé de préparation de composés type azoïque.

Les composés de type azoïque, et notamment le 2,2'-azobis (isobutyronitrile), sont des produits bien connus qui sont utilisés notamment comme agent gonflant ou intermédiaire de synthèse ou initiateur des réactions de polymérisation mettant en oeuvre des radicaux libres.

Ces réactions peuvent être des réactions de polymérisation en masse, en solution, en suspension ou en émulsion et faire appel à des monomères très variés par exemple (méth)acryliques, vinyliques tels que l'acrylamide, l'acrylonitrile, le (méth)acrylate d'alkyle, le styrène, l'acétate et le chlorure de vinyle, le chlorure de vinylidène. Les domaines d'application sont donc très divers et concernent notamment (mais non exclusivement) les feuilles ou fibres acryliques, les floculants, les peintures, les résines d'enduction, les polyols greffés, le polystyrène, le PVC, le PVA, le PMMA.

Les composés azoïques sont en général obtenus en oxydant les dérivés hydrazoïques correspondants (US 2 469 358 ; US 2 586 995). Après oxydation, la suspension résultante est essorée, puis séchée pour donner un solide sous forme de poudre de granulométrie moyenne généralement comprise entre 20 et 110 µm. Par exemple, le 2,2'-azobis(isobutyronitrile) obtenu en faisant réagir le dérivé hydrazoïque correspondant avec le chlore, dans un milieu aqueux, se présente sous forme de poudre de granulométrie moyenne d'environ 45 µm après séchage et la granulométrie à 10 % en poids (d10) est d'environ 20 µm.

Or les composés azoïques, sous forme telle que décrite précédemment, posent de nombreux problèmes :
- ils génèrent des poussières susceptibles de présenter un risque d'explosion et/ou d'hygiène industrielle,
- ils présentent une mauvaise aptitude à la coulabilité
- et des problèmes de mottage sont souvent rencontrés lors de leur stockage.

L'invention permet de résoudre en partie ou en totalité les problèmes identifiés ci-dessus. L'invention fournit une solution globale permettant d'augmenter la granulométrie moyenne d'un composé azoïque par rapport à celle d'un composé obtenu par un procédé de fabrication classique. La granulométrie moyenne peut être même doublée voir triplée.

L'invention fournit donc un procédé de fabrication d'un composé azoïque (A), à partir du composé hydrazoïque (HA) correspondant, par ensemencement.

Le procédé selon l'invention comprend une étape dans laquelle on fait réagir un oxydant avec un composé hydrazoïque (HA), dans un milieu liquide, en présence d'une quantité suffisante de cristaux du composé azoïque correspondant ( semences).

Selon un mode de réalisation, le composé hydrazoïque est en suspension dans le milieu liquide.

Selon un mode de réalisation, le composé hydrazoïque est en émulsion dans le milieu liquide.

Cette étape d'oxydation peut être schématisée par l'équation suivante : 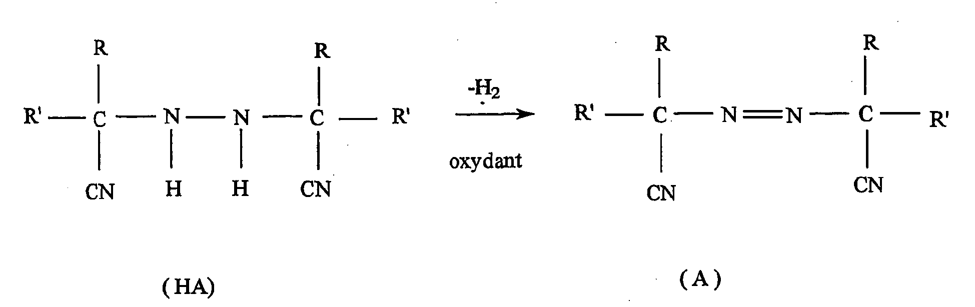 avec R et R' dans (HA) et (A) pouvant être identiques ou différents, et représentant chacun :
- un groupe alkyle linéaire ou ramifié, de préférence un groupe C₁-C₆ alkyle, éventuellement substitué par un groupe hydroxy, alkoxy, carboxy ou par un atome d'halogène, ou
- un groupe cycloalkyle ayant de préférence de 3 à 6 atomes de carbone, éventuellement substitué par un groupe hydroxy, alkoxy, carboxy ou par un atome d'halogène, ou
- un groupe aryle tel que phényle ou naphtyle, éventuellement substitué par un groupe hydroxy, alkyl, alkoxy, carboxy ou par un atome d'halogène, ou
- un groupe aralkyle, tel que benzyle, phénéthyle, éventuellement substitué par un ou plusieurs groupes alkyle, alkoxy, hydroxy, carboxy, ou par un ou plusieurs atomes d'halogène ; ou bien R et R' forment avec l'atome de carbone auquel il est (ou ils sont) reliée(s), un radical cycloalkyle.

A titre d'exemple de composé (A) on peut citer le 2,2' azobis isobutyronitrile (R = R' = CH₃), le 2,2'-azobis(2,4-diméthyl-valéronitrile), le 2,2'-azobis(2-méthylbutyronitrile) , le 1,1'-azobis(1-cyclohexanecarbonitrile) et le 4,4'-azobis(4-cyanopentanoic acid).

A titre d'exemple d'oxydant on peut citer le chlore, l'oxygène, le peroxyde d'hydrogène et l'ozone.

De préférence, le milieu liquide est aqueux ( c'est à dire constituée essentiellement d'eau).

Outre les réactifs ( HA et oxydant ), le milieu réactionnel liquide peut comprendre un catalyseur , comme par exemple les ions bromés. Il peut également comprendre des additifs, tels que les agents tensioactifs comme par exemple les bis sulfosuccinate de sodium, notamment le bis(2-éthylhexyl)sulfosuccinate de sodium.

La température du milieu réactionnel est généralement voisine de la température ambiante. Elle est de préférence comprise entre 15 et 25 ° C et avantageusement comprise entre 18 et 20° C .

Les semences ou cristaux du composé ( A ) peuvent être présents dans le milieu réactionnel avant le début de la réaction d'oxydation et/ou introduits pendant la réaction d'oxydation.

Après l'étape d'oxydation, la suspension résultante est essorée puis le solide obtenu est lavé et enfin séché.

Selon un mode de réalisation préférée de l'invention, on oxyde le dihydrocitogène ( R=R'=CH₃), en suspension dans un milieu aqueux, par du chlore en présence d'une quantité suffisante de cristaux du 2,2' azobis(isobutyronitrile) (AZDN) de granulométrie moyenne, avantageusement comprise entre 110 et 180 µm et mieux encore comprise entre 110 et 150 µm.

De préférence, ces cristaux d'AZDN (semences ) sont présents en début de l'étape d'oxydation et représentent entre 0,5 et 20 % en poids, avantageusement entre 5 et 15 % en poids par rapport au milieu réactionnel (c'est à dire eau + réactifs + additifs + semences ).

Le dihydrocitogène mis en suspension dans le milieu aqueux représente de préférence entre 2 et 30 % en poids , et avantageusement entre 5 -15 % en poids par rapport au milieu réactionnel (c'est-à-dire eau + réactifs + additifs + semences).

Le milieu réactionnel peut être agité à l'aide de tout moyen connu.

La température du milieu réactionnel est de préférence comprise entre 15 et 25°C et avantageusement comprise entre 18 et 20° C.

La durée de l'étape d'oxydation selon l'invention varie suivant la granulométrie souhaitée. Elle est de préférence comprise entre 2 et 6 heures.

De préférence, le chlore est injecté dans le milieu aqueux, en continu, sous forme gazeux, pendant toute la durée de l'oxydation.

A l'issue de l'étape d'oxydation selon l'invention, le produit obtenu est essoré, puis lavé et enfin séché pour donner de l'AZDN de granulométrie moyenne souhaitée.

Les cristaux d'AZDN (semences) peuvent être préparées à partir du dihydrocitogène par un procédé d'oxydation classique (poc), suivi d'une étape (r1) dans laquelle on fait réagir le dihydrocitogène avec du chlore dans un milieu aqueux comprenant une partie de la suspension provenant du procédé classique (poc).

Suivant la granulométrie moyenne d'AZDN souhaitée, on peut de nouveau faire réagir (r2) le dihydrocitogène avec le chlore dans un milieu aqueux comprenant une partie de la suspension provenant de l'étape précédente ( r1 ).

Avantageusement, les semences peuvent être obtenues en faisant réagir n fois, n étant un nombre entier supérieur à 2 , de préférence allant de 3 à 5, le dihydrocitogène avec le chlore dans un milieu aqueux comprenant à chaque fois une partie de la suspension provenant de la réaction d'oxydation qui précède.

De préférence, le milieu aqueux pour l'étape réactionnelle ( m) comprend la moitié de la quantité de la suspension résultante de l'étape réactionnelle (r(n-1)).

La préparation des semences d'AZDN décrite ci-dessus peut être appliquée à d'autres composés azoïques A, oxydants et milieu liquide.

Lorsque la granulométrie moyenne des semences destinées à l'étape d'oxydation selon l'invention est atteinte, il n'est pas nécessaire de procéder comme décrite ci-dessus pour fabriquer de nouvelles semences. Il suffit après chaque batch d'oxydation de vidanger seulement une partie de la suspension résultante et d'en laisser le reste pour l'étape d'oxydation suivante. Ceci permet de s'affranchir des semences de granulométrie intermédiaire.

On peut également utiliser comme semences, les cristaux de composé A obtenus après essorage d'une suspension résultant de l'étape d'oxydation selon l'invention, suivi éventuellement d'un séchage.

La demanderesse a constaté que le composé A obtenu après l'étape d'oxydation décrite ci-dessus facilite les opérations ultérieures d'essorage et de séchage et permet ainsi d'augmenter la productivité de l'installation de fabrication. La granulométrie du composé A séché est moins étalée et le composé A séché présente moins de poussières et offre une meilleure coulabilité.

### PARTIE EXPERIMENTALE

### Préparation des semences

### Essai 1

Dans un réacteur de capacité 1,5 I muni d'un système d'agitation permettant le brassage d'une suspension, on introduit successivement :
- 216 g ( 1,3 moles) du dihydrocitogène sec
- 1350 g d'eau
- 0,1 g de di(2 éthylhexyl)sulfosuccinate (DOS)
- 0,6 g de bromure de sodium.

On introduit ensuite en continu, à l'aide d'une arrivée de gaz plongeant dans le réacteur, dans le milieu aqueux ainsi formé, 95 g de chlore sur une durée totale de 5,5 heures. Au cours de la réaction, le milieu est maintenu à une température comprise entre 18 à 20° C. On arrête l'agitation une heure après la fin de l'introduction du chlore.

On filtre ensuite la suspension, puis on lave le solide obtenu jusqu'à l'obtention d'un pH des eaux de lavage voisin de 7.

Enfin on sèche sous vide à 30° C pendant 12 heures le solide lavé pour atteindre une humidité finale inférieure à 0,05% en poids. Le solide séché (cristaux de l'azobisisobutyronitrile ( AZDN )) a une granulométrie moyenne ( d50 ) de 45 µm et une granulométrie ( d10) de 20 µm. La coulabilité de ce solide est très mauvaise.

### Essai 2

On opère comme décrit à l'essai 1, sauf que l'on ajoute 25 g de solide séché provenant de l'essai 1 avant d'introduire le chlore.

On obtient après séchage des cristaux de granulométrie moyenne ( d50 ) de 75 µm et un d10 de 40 µm.

### Essai 2 bis

On opère comme décrit à l'essai 2 sauf que l'on utilise 216 g (au lieu de 25) de cristaux d'AZDN provenant de l'essai 1.

On obtient après séchage des cristaux de granulométrie moyenne ( d50 ) de 60 µm et un d10 de 35 µm.

### Essai 3

On opère comme décrit à l'essai 2 sauf que l'on ajoute 25 g de solide séché provenant de l'essai 2 à la place du solide provenant de l'essai 1.

On obtient après séchage des cristaux de granulométrie moyenne ( d50) de 85 µm.

### Essai 4

On opère comme décrit à l'essai 1 et à la fin de la réaction on vidange seulement la moitié de la suspension obtenue. On introduit alors successivement dans le réacteur contenant l'autre moitié :
- 216 g ( 1,3 moles) du dihydrocitogène sec
- 675 g d'eau
- 0,1 g de di(2 éthylhexyl)sulfosuccinate (DOS)
- 0,3g de bromure de sodium.

On introduit ensuite en continu, à l'aide d'une arrivée de gaz plongeant dans le réacteur, dans le milieu aqueux ainsi formé, 95 g de chlore sur une durée totale de 5,5 heures. Au cours de la réaction, le milieu est maintenu à une température comprise entre 18 à 20° C. On arrête l'agitation une heure après la fin de l'introduction du chlore, puis on vidange à nouveau seulement la moitié de la suspension obtenue.

Ensuite, on introduit successivement dans le réacteur contenant l'autre moitié restante, les différents constituants de la charge indiquée ci-dessus. Après l'arrêt de la réaction, on vidange que la moitié de la suspension obtenue et on introduit dans le réacteur contenant l'autre moitié la même charge que pour le batch précédent.

Après deux demi-vidanges supplémentaires (comme indiqué ci-dessus) la moitié de la suspension finale est utilisée pour effectuer l'exemple 1 et l'autre moitié est essorée, puis le solide est lavé jusqu'à l'obtention d'un pH des eaux de lavage voisin de 7 et enfin le solide séché est lavé pour donner des cristaux de granulométrie moyenne (d50) de 150 µm et une granulométrie ( d10) de 60 µm.

### Exemple 1

On introduit successivement dans le réacteur contenant la moitié de la suspension finale provenant de l'essai 4:
- 216 g ( 1,3 moles) du dihydrocitogène sec
- 675 g d'eau
- 0,1 g de di(2 éthylhexyl)sulfosuccinate (DOS)
- 0,3g de bromure de sodium.

On introduit ensuite en continu, à l'aide d'une arrivée de gaz plongeant dans le réacteur, dans le milieu aqueux ainsi formé, 95 g de chlore sur une durée totale de 5,5 heures. Au cours de la réaction, le milieu est maintenu à une température comprise entre 18 à 20° C. On arrête l'agitation une heure après la fin de l'introduction du chlore et on filtre la suspension. Le solide obtenu est ensuite filtré, puis lavé à l'eau jusqu'à l'obtention d'un pH des eaux de lavage voisin de 7. Par rapport à l'essai 1, la durée de filtration est réduite de 50%.

L'humidité résiduelle du solide lavé est de 30% inférieure par rapport à celle du solide obtenu suivant l'essai 1 et la densité apparente est augmentée de 30%.

Enfin on sèche sous vide à 30° C, pendant 6 heures, le solide lavé pour atteindre une humidité finale inférieure à 0,05% en poids. Le solide séché ( cristaux de l'azobisisobutyronitrile ( AZDN )) a une granulométrie moyenne ( d50 ) de 150 µm et une granulométrie ( d10) est de 60 µm.

### Exemple 2

On opère comme décrit à l'exemple 1 sauf que la durée d'introduction du chlore est de 4 heures.

Un produit similaire à celui de l'exemple 1 est obtenu.

### Exemple 3

On opère comme décrit à l'exemple 1 sauf que la vitesse d'agitation est augmentée de 50%.

Un produit similaire à celui de l'exemple 1 est obtenu.

### Exemple 4

Les conditions opératoires de l'exemple 1 ont été reproduits à l'échelle industrielle dans un réacteur de 5 m³. Un produit similaire à celui de l'exemple 1 est obtenu.

### Exemple C1

On opère comme décrit à l'essai 1 sauf que le chlore est introduit sur une durée de 4 heures ( au lieu de 5,5 ).

Le solide séché ( cristaux de l'azobisisobutyronitrile ( AZDN )) a une granulométrie moyenne ( d50 ) inférieure à celle de l'essai 1.

### Exemple C2

On opère comme décrit à l'essai 1 sauf que la vitesse d'agitation est augmentée de 50%.

On obtient une granulométrie moyenne inférieure à celle des cristaux de l'essai 1.

### Exemple 5

On opère comme décrit à l'essai 1 sauf que l'on introduit dans le réacteur, avant le début de la chloration, 216 g de cristaux d'AZDN de granulométrie moyenne de 150 µm, obtenus à l'exemple 1.

Après séchage, on obtient des cristaux d'AZDN de granulométrie moyenne (d50) de 150 µm.

## Revendications

1. Procédé de fabrication d'un composé azoïque (A) comprenant une étape dans laquelle on fait réagir ( suivant l'équation ci-dessous) un oxydant avec un composé hydrazoïque (HA) , dans un milieu liquide, en présence d'une quantité suffisante de cristaux du composé azoïque correspondant (semences) : 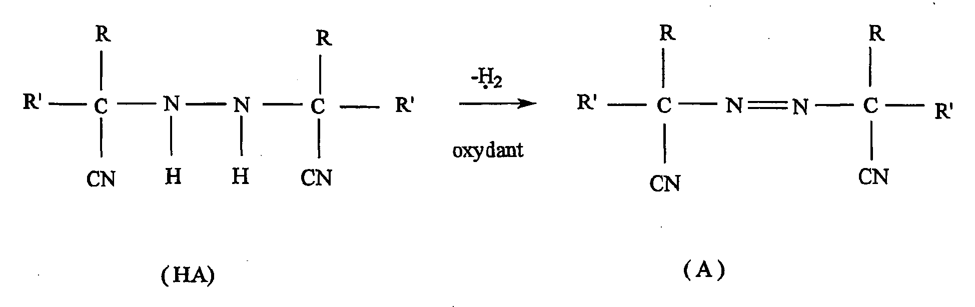 avec R et R' dans (HA) et (A) pouvant être identiques ou différents, et représentant chacun :
- un groupe alkyle linéaire ou ramifié, de préférence un groupe C₁-C₆ alkyle, éventuellement substitué par un groupe hydroxy, alkoxy, carboxy ou par un atome d'halogène, ou
- un groupe cycloalkyle ayant de préférence de 3 à 6 atomes de carbone, éventuellement substitué par un groupe hydroxy, alkoxy, carboxy ou par un atome d'halogène, ou
- un groupe aryle tel que phényle ou naphtyle, éventuellement substitué par un groupe hydroxy, alkyl, alkoxy, carboxy ou par un atome d'halogène, ou
- un groupe aralkyle, tel que benzyle, phénéthyle, éventuellement substitué par un ou plusieurs groupes alkyle, alkoxy, hydroxy, carboxy, ou par un ou plusieurs atomes d'halogène ; ou bien R et R' forment avec l'atome de carbone auquel il est (ou ils sont) reliée(s), un radical cycloalkyle.

2. Procédé selon la revendication 1 **caractérisé en ce que** le composé A est le 2,2' azobis isobutyronitrile (R = R' = CH₃), le 2,2'-azobis(2,4-diméthyl-valéronitrile), le 2,2'-azobis(2-méthylbutyronitrile) , le 1,1'-azobis(1-cyclohexanecarbonitrile) ou le 4,4'-azobis(4-cyanopentanoic acid).

3. Procédé selon la revendication 1 ou 2 **caractérisé en ce que** l'oxydant est le chlore, l'oxygène, le peroxyde d'hydrogène ou l'ozone.

4. Procédé selon l'une des revendications 1 à 3 **caractérisé en ce que** le milieu liquide est aqueux.

5. Procédé de fabrication de 2,2' azobis(isobutyronitrile) à partir du dihydrocitogène **caractérisé en ce que** l'on oxyde le dihydrocitogène, en suspension dans un milieu aqueux, par du chlore en présence d'une quantité suffisante de cristaux de 2,2' azobis(isobutyronitrile) de granulométrie moyenne, avantageusement comprise entre 110 et 180 µm et mieux encore comprise entre 110 et 150 µm.

6. Procédé selon la revendication 5 **caractérisé en ce que** les cristaux d'AZDN (semences ) sont présents en début de l'étape d'oxydation et représentent entre 0,5 et 20 % en poids, avantageusement entre 5 et 15 % en poids par rapport au milieu réactionnel.

7. Procédé selon la revendication 5 ou 6 **caractérisé en ce que** le dihydrocitogène mis en suspension dans le milieu aqueux représente entre 2 et 30 % en poids , et avantageusement entre 5 - 15 % en poids par rapport au milieu réactionnel.

8. Procédé selon l'une quelconque des revendications précédentes **caractérisé en ce que** la température du milieu réactionnel est comprise entre 15 et 25°C et avantageusement comprise entre 18 et 20° C.

9. Procédé selon l'une quelconque des revendications précédentes **caractérisé en ce qu'**après l'étape d'oxydation, la suspension résultante est essorée puis lavé et séché.

10. Procédé selon l'une quelconque des revendications précédentes **caractérisé en ce que** les cristaux de composé (A) peuvent être préparés à partir du composé HA correspondant par un procédé d'oxydation classique suivi de n réactions, n étant un nombre entier supérieur à 2, de préférence allant de 3 à 5, au cours desquelles le composé HA est oxydé dans un milieu liquide comprenant une partie de la suspension provenant de la réaction qui précède.

## Claims

1. Method of manufacture of an azo compound (A) comprising a step in which an oxidizing agent is reacted (in accordance with the equation given below) with a hydrazo compound (HA), in a liquid medium, in the presence of a sufficient quantity on crystals of the corresponding azo compound (seeds): 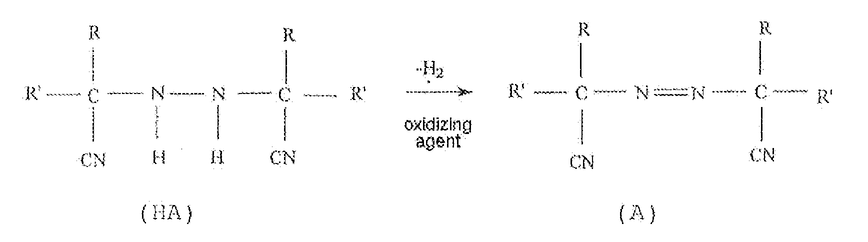 where R and R' in (HA) and (A) can be identical or different, with each representing:
- a linear or branched alkyl group, preferably a C₁-C₆ alkyl group, possibly substituted by a hydroxy, alkoxy or carboxy group or by a halogen atom, or
- a cycloalkyl group preferably with 3 to 6 carbon atoms, possibly substituted by a hydroxy, alkoxy or carboxy group or by a halogen atom, or
- an aryl group such as phenol or naphthyl, possibly substituted by a hydroxy, alkyl, alkoxy or carboxy group or by a halogen atom, or
- an aralkyl group such as benzyl or phenethyl, possibly substituted by one or more alkyl, alkoxy, hydroxy or carboxy groups, or by one or more halogen atoms; or alternatively R and R' form, with the carbon atom to which it is (or they are) joined, a cycloalkyl radical.

2. Method as claimed in claim 1, **characterized in that** compound A is 2,2' azobis isobutyronitrile (R = R' = CH₃), 2,2'-azobis(2,4-dimethyl-valeronitrile), 2,2'-azobis(2-methylbutyronitrile), 1,1'-azobis(1-cyclo-hexanecarbonitrile) or 4,4'-azobis(4-cyanopentanoic acid).

3. Method as claimed in claim 1 or 2, **characterized in that** the oxidizing agent is chlorine, oxygen, hydrogen peroxide or ozone.

4. Method as claimed in one of the claims 1 to 3, **characterized in that** the liquid medium is aqueous.

5. Method of manufacture of 2,2'-azobis(isobutyronitrile) starting from dihydrocitogen, **characterized in that** dihydrocitogen, in suspension in an aqueous medium, is oxidized with chlorine in the presence of a sufficient quantity of crystals on 2,2'-azobis(isobutyronitrile) with average grain size advantageously between 110 and 180 µm and better still between 110 and 150 µm.

6. Method as claimed in claim 5, **characterized in that** the crystals of AIBN (seeds) are present at the start of the oxidation step and represent between 0.5 and 20 wt.%, advantageously between 5 and 15 wt.%, relative to the reaction medium.

7. Method as claimed in claim 5 or 6, **characterized in that** the dihydrocitogen suspended in the aqueous medium represents s between 2 and 30 wit.%, and advantageously between 5 and 15 wt.% relative to the reaction medium.

8. Method as claimed in any one of the preceding claims, **characterised in that** the temperature of the reaction medium is between 15 and 25°C and advantageously between 18 and 20°C.

9. Method as s claimed in any one of the preceding claims, **characterized in that** after the oxidation step, the resulting suspension is drained then washed and dried.

10. Method as claimed in any one of the preceding claims, **characterized in that** the crystals of compound (A) can be prepared starting from the corresponding compound HA by a conventional method of oxidation followed by n reactions, n being an integer greater than 2, preferably in the rang from 3 to 5, in the course of which compound HA is oxidized in a Liquid medium containing a proportion of the suspension resulting from the preceding reaction.

## Patentansprüche

1. Verfahren zur Herstellung einer Azoverbindung (A) mit einem Schritt, bei dem man (gemäß der folgenden Reaktionsgleichung) ein Oxidationsmittel in einem flüssigen Medium in Gegenwart einer ausreichenden Menge von Kristallen der entsprechenden Azoverbindung (Keimen) mit einer Hydrazoverbindung (HA) umsetzt: 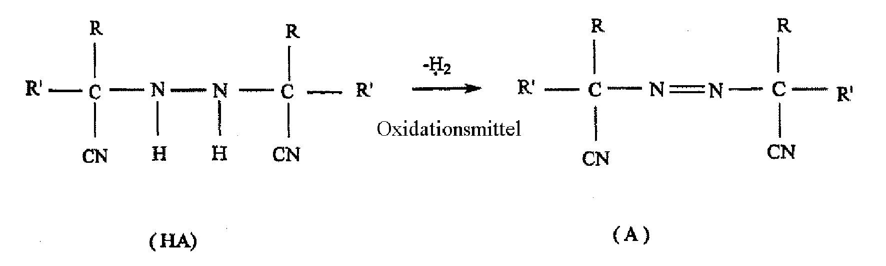 wobei R und R' in (HA) und (A) gleich oder verschieden sein können und jeweils für
- eine lineare oder verzweigte Alkylgruppe, vorzugsweise eine C₁-C₆-Alkylgruppe, die gegebenenfalls durch eine Hydroxy-, Alkoxy- oder Carboxygruppe oder durch ein Halogenatom substituiert ist, oder
- eine Cycloalkylgruppe mit vorzugsweise 3 bis 6 Kohlenstoffatomen, die gegebenenfalls durch eine Hydroxy-, Alkoxy- oder Carboxygruppe oder durch ein Halogenatom substituiert ist, oder
- eine Arylgruppe wie Phenyl oder Naphthyl, die gegebenenfalls durch eine Hydroxy-, Alkyl-, Alkoxy- oder Carboxygruppe oder durch ein Halogenatom substituiert ist, oder
- eine Aralkylgruppe wie Benzyl oder Phenethyl, die gegebenenfalls durch eine oder mehrere Alkyl-, Alkoxy-, Hydroxy- oder Carboxygruppen oder durch ein oder mehrere Halogenatome substituiert ist, stehen oder R und R' auch mit dem Kohlenstoffatom, an das es gebunden ist bzw. sie gebunden sind, einen Cycloalkylrest bilden.

2. Verfahren nach Anspruch 1, **dadurch gekennzeichnet, daß** es sich bei der Verbindung A um 2,2'-Azobisisobutyronitril (R = R' = CH₃), 2,2'-Azobis(2,4-dimethylvaleronitril), 2,2'-Azobis(2-methylbutyronitil), 1,1'-Azobis(cyclohexancarbonitril) oder 4,4'-Azobis(4-cyanopentansäure) handelt.

3. Verfahren nach Anspruch 1 oder 2, **dadurch gekennzeichnet, daß** es sich bei dem Oxidationsmittel um Chlor, Sauerstoff, Wasserstoffperoxid oder Ozon handelt.

4. Verfahren nach einem der Ansprüche 1 bis 3, **dadurch gekennzeichnet, daß** das flüssige Medium wäßrig ist.

5. Verfahren zur Herstellung von 2,2'-Azobis-(isobutyronitril) aus Dihydrocitogen, **dadurch gekennzeichnet, daß** man das Dihydrocitogen in Suspension in wäßrigem Medium in Gegenwart einer ausreichenden Menge von Kristallen von 2,2'-Azobis(isobutyronitril) mittlerer Teilchengröße, vorteilhafterweise zwischen 110 und 180 µm und noch besser zwischen 110 und 150 µm, mit Chlor oxidiert.

6. Verfahren nach Anspruch 5, **dadurch gekennzeichnet, daß** die AIBN-Kristalle (Keime) zu Beginn des Oxidationsschritts vorliegen und zwischen 0,5 und 20 Gew.-%, vorteilhafterweise zwischen 5 und 15 Gew.-%, bezogen auf das Reaktionsmedium, ausmachen.

7. Verfahren nach Anspruch 5 oder 6, **dadurch gekennzeichnet, daß** das in dem wäßrigen Medium suspendierte Dihydrocitogen zwischen 2 und 30 Gew.-% und vorteilhafterweise zwischen 5 und 15 Gew.-%, bezogen auf das Reaktionsmedium, ausmacht.

8. Verfahren nach einem der vorhergehenden Ansprüche, **dadurch gekennzeichnet, daß** die Temperatur des Reaktionsmediums zwischen 15 und 25°C und vorteilhafterweise zwischen 18 und 20°C liegt.

9. Verfahren nach einem der vorhergehenden Ansprüche, **dadurch gekennzeichnet, daß** die erhaltene Suspension nach dem Oxidationsschritt abgeschleudert und dann gewaschen und getrocknet wird.

10. Verfahren nach einem der vorhergehenden Ansprüche, **dadurch gekennzeichnet, daß** die Kristalle von Verbindung (A) aus der entsprechenden Verbindung HA durch ein herkömmliches Oxidationsverfahren gefolgt von n Reaktionen, wobei n für eine ganze Zahl von mehr als 2, vorzugsweise im Bereich von 3 bis 5, steht, in deren Verlauf die Verbindung HA in einem flüssigen Medium, das einen Teil der Suspension aus der vorhergehenden Reaktion enthält, oxidiert wird, hergestellt werden können.
